# EUROPEAN PATENT APPLICATION

(11) **EP 3 977 997 A1**
(43) Date of publication of application: **06.04.2022**
(21) Application number: 20812881.9
(22) Date of filing: 29.05.2020
(51) Int. Cl.: A61K 31/50, A61K 47/38, A61K 9/26, A61P 19/02, A61K 47/14

(54) **SOLID DISPERSION AND PREPARATION METHOD THEREFOR**

(30) Priority: 31.05.2019 CN 201910468254
(71) Applicant: Jiangsu Hengrui Medicine Co., Ltd., Lianyungang, Jiangsu 222047 (CN)
(72) Inventor: ZHOU, Xianqiang, Lianyungang, Jiangsu 222047 (CN); DU, Zhenxing, Lianyungang, Jiangsu 222047 (CN); WANG, Jie, Lianyungang, Jiangsu 222047 (CN)
(74) Representative: Regimbeau
(86) International application number: PCT/CN2020/093206
(87) International publication number: WO 2020/239065

(57) **Abstract**

Solid dispersion and a preparation method therefor. In a specific embodiment, the solid dispersion contains an active ingredient (*R*)-4-amino-1-(1-(but-2-ynyl)pyrrolidin-3-yl))-3-(4-(2,6-difluorophenoxy)phenyl)-1,6-dihydro-7H-pyrrolo[2,3-d]pyridazin-7-one or a salt thereof, and a carrier material, and the pH value is adjusted; employing a method that adds an appropriate amount of acid effectively inhibits an emulsification phenomenon in a reverse solvent process, thereby obtaining solid dispersion having a moderate particle size and uniform content.

## Description

### TECHNICAL FIELD

The present disclosure belongs to the field of pharmaceutical preparations, and specifically relates to a solid dispersion, a method for preparing the same and a use thereof.

### BACKGROUND

The compound of formula I (*R*)-4-amino-1-(1-(but-2-ynoyl)pyrrolidin-3-yl)-3-(4-(2,6-difluorophenoxy)phenyl)-1,6-dihydro-7*H*-pyrrolo[2,3-d]pyridazin-7-one is a BTK inhibitor with good target specificity and high selectivity for kinase. It can inhibit BTK phosphorylation and down-regulate BCR signal transduction pathway, thereby selectively inhibiting the proliferation and migration of B cell tumor. Recent clinical trials have shown that it has excellent pharmacodynamic activity,

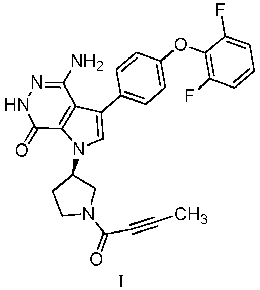

The compound of formula I is an active substance with low solubility, and its druggability needs to be studied in depth and solved by pharmaceutical researchers. WO2019007317 discloses a solid dispersion preparation containing the compound of formula I, which applies solid dispersion technology to solve the problem of dissolution after the compound is formulated as a drug. The methods for preparing solid dispersion include melting method, solvent method, solvent-melting method, solvent-spray drying method or grinding method. The anti-solvent method is the mildest method for preparing solid dispersion, which avoids subjecting active substance to high temperature, and is suitable for various heat-labile or volatile drugs, and is easy to conduct.

However, during the preparation of solid dispersion, especially when the anti-solvent method is used, emulsification often occurs, which will affect the preparation of solid dispersion, resulting in uneven content of active ingredient between batches, thereby affecting the effectiveness and safety.

Common demulsification methods include static method, high-voltage electric field method, chemical demulsification method (such as salt fractionation method, coagulation method, salt fractionation-coagulation method), centrifugal method, ultrafiltration method and vacuum separation method. Each demulsification method has its own advantages, while accompanied by its own disadvantages. The chemical demulsification method such as the salt fractionation method needs to introduce an inorganic salt as an additional chemical reagent into the emulsification system, thereby increasing the cost of subsequent processing. At the same time, the selection of demulsification method also needs to consider the characteristics of the product itself.

### SUMMARY OF THE INVENTION

The present disclosure provides a method for preparing a solid dispersion, comprising the steps of dissolving a carrier material and an active ingredient in a good solvent to obtain a solution, adding the resulting solution to a poor solvent, and adjusting the pH to 1.0 to 6.0; or, comprising the steps of dissolving a carrier material and an active ingredient in a good solvent to obtain a solution, and adding the resulting solution to a poor solvent, wherein the pH of the poor solvent is 1.0 to 6.0.

In alternative embodiments, the pH value can be 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0 or any value between any two values, and preferably the pH is 2.0 to 4.0.

Controlling the pH of the poor solvent or the crystallization solution to be less than 6.0 can effectively avoid the aforementioned emulsification problem during the anti-solvent method. There is no need to conduct additional demulsification methods such as salt fractionation method and coagulation method, which facilitates subsequent filtration or washing process, and provides samples with even active ingredient content between batches.

Furthermore, the method of the present disclosure also comprises a step of filtration, washing or drying.

In some embodiments, the good solvent is at least one selected from the group consisting of dimethyl sulfoxide, N,N-dimethylformamide, N,N-dimethylacetamide, acetone, ethanol, tetrahydrofuran and methanol, and preferably N,N-dimethylformamide or N,N-dimethylacetamide; and the poor solvent is at least one selected from the group consisting of diethyl ether, *n*-hexane, petroleum ether and water, such as a mixed solution of water with one or more of ether, *n*-hexane and petroleum ether.

In some embodiments, the carrier material and the active ingredient or the pharmaceutically acceptable salt thereof are dissolved in the good solvent of N,N-dimethylformamide, and the resulting solution is added to the poor solvent of water, wherein the pH of the poor solvent of water is 1.0 to 6.0.

In some embodiments, the carrier material and the active ingredient or the pharmaceutically acceptable salt thereof are dissolved in the good solvent of N,N-dimethylacetamide, and the resulting solution is added to the poor solvent of water, wherein the pH of the poor solvent of water is 1.0 to 6.0.

In some embodiments, the carrier material and the active ingredient or the pharmaceutically acceptable salt thereof are dissolved in the good solvent of dimethyl sulfoxide, and the resulting solution is added to the poor solvent of water, wherein the pH of the poor solvent of water is 1.0 to 6.0.

Furthermore, the reagent used to adjust pH is at least one selected from the group consisting of hydrochloric acid, sulfuric acid, acetic acid and phosphoric acid, and preferably sulfuric acid or hydrochloric acid.

In another aspect, the rate of the dropwise addition (that is, the addition of the good solvent to the poor solvent) will also affect the quality of crystallization to a certain extent, such as the size and uniformity of the precipitated solid particles.

In some embodiments, the rate of the dropwise addition (the addition of the good solvent to the poor solvent) can be 1 to 2500 g/min, such as 1 to 2250 g/min, 1 to 2000 g/min. The rate of the dropwise addition can be adjusted according to the needs of preparation.

In other embodiments, the addition to the poor solvent of the present disclosure is accompanied by a stirring process, the stirring rate is selected from the group consisting of 20 to 1000 rpm, non-limiting examples include 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 rpm or any value between any two values, and preferably 100 to 600 rpm.

The method for preparing a solid dispersion of the present disclosure can be applied to any insoluble solids. In alternative embodiments, the active ingredient is (*R*)-4-amino-1-(1-(but-2-ynoyl)pyrrolidin-3-yl)-3-(4-(2,6-difluorophenoxy)phenyl)-1,6-dihydro-7H-pyrrolo[2,3-d]pyridazin-7-one or the pharmaceutically acceptable salt thereof.

Furthermore, the carrier material is at least one selected from the group consisting of hydroxypropyl methylcellulose succinate, polyvinylpyrrolidone, hydroxypropyl methylcellulose phthalate and polyvinylpyrrolidone.

In some embodiments, the carrier material hydroxypropyl methylcellulose acetate succinate and the active ingredient are dissolved in the good solvent of N,N-dimethylformamide, and the resulting solution is added to the poor solvent of water, wherein the pH of the poor solvent of water = 1.0 to 6.0.

In some embodiments, the carrier material of hydroxypropyl methylcellulose acetate succinate and the active ingredient are dissolved in the good solvent N,N-dimethylacetamide, and the resulting solution is added to the poor solvent of water, wherein the pH of the poor solvent of water is 1.0 to 6.0.

In some embodiments, the carrier material of hydroxypropyl methylcellulose acetate succinate and the active ingredient are dissolved in the good solvent of dimethyl sulfoxide, and the resulting solution is added to the poor solvent of water, wherein the pH of the poor solvent of water is 1.0 to 6.0.

In some embodiments, the carrier material of polyvinylpyrrolidone and the active ingredient are dissolved in the good solvent of N,N-dimethylformamide, and the resulting solution is added to the poor solvent of water, wherein the pH of the poor solvent of water is 1.0 to 6.0.

In some embodiments, the carrier material of polyvinylpyrrolidone and the active ingredient are dissolved in the good solvent of N,N-dimethylacetamide, and the resulting solution is added to the poor solvent of water, wherein the pH of the poor solvent of water is 1.0 to 6.0.

In some embodiments, the carrier material of polyvinylpyrrolidone and the active ingredient are dissolved in the good solvent of dimethyl sulfoxide, and the resulting solution is added to the poor solvent of water, wherein the pH of the poor solvent of water is 1.0 to 6.0.

In some embodiments, the carrier material of hydroxypropyl methylcellulose phthalate and the active ingredient are dissolved in the good solvent of N,N-dimethylformamide, and the resulting solution is added to the poor solvent of water, wherein the pH of the poor solvent of water is 1.0 to 6.0.

In some embodiments, the carrier material of hydroxypropyl methylcellulose phthalate and the active ingredient are dissolved in the good solvent of N,N-dimethylacetamide, and the resulting solution is added to the poor solvent of water, wherein the pH of the poor solvent of water is 1.0 to 6.0.

In some embodiments, the carrier material of hydroxypropyl methylcellulose phthalate and the active ingredient are dissolved in the good solvent of dimethyl sulfoxide, and the resulting solution is added to the poor solvent of water, wherein the pH of the poor solvent of water is 1.0 to 6.0.

In some embodiments, the carrier material of hydroxypropyl methylcellulose acetate succinate and the active ingredient are dissolved in the good solvent of N,N-dimethylformamide, the resulting solution is added to the poor solvent of water, and the pH is adjusted to 1.0 to 6.0.

In some embodiments, the carrier material of hydroxypropyl methylcellulose acetate succinate and the active ingredient are dissolved in the good solvent of N,N-dimethylacetamide, the resulting solution is added to the poor solvent of water, and the pH is adjusted to 1.0 to 6.0.

In some embodiments, the carrier material of hydroxypropyl methylcellulose acetate succinate and the active ingredient are dissolved in the good solvent of dimethyl sulfoxide, the resulting solution is added to the poor solvent of water, and the pH is adjusted to 1.0 to 6.0.

In some embodiments, the carrier material of polyvinylpyrrolidone and the active ingredient are dissolved in the good solvent of N,N-dimethylformamide, the resulting solution is added to the poor solvent of water, and the pH is adjusted to 1.0 to 6.0.

In some embodiments, the carrier material of polyvinylpyrrolidone and the active ingredient are dissolved in the good solvent of N,N-dimethylacetamide, the resulting solution is added to the poor solvent of water, and the pH is adjusted to 1.0 to 6.0.

In some embodiments, the carrier material of polyvinylpyrrolidone and the active ingredient are dissolved in the good solvent of dimethyl sulfoxide, the resulting solution is added to the poor solvent of water, and the pH is adjusted to 1.0 to 6.0.

In some embodiments, the carrier material of hydroxypropyl methylcellulose phthalate and the active ingredient are dissolved in the good solvent of N,N-dimethylformamide, the resulting solution is added to the poor solvent of water, and the pH is adjusted to 1.0 to 6.0.

In some embodiments, the carrier material of hydroxypropyl methylcellulose phthalate and the active ingredient are dissolved in the good solvent of N,N-dimethylacetamide, the resulting solution is added to the poor solvent of water, and the pH is adjusted to 1.0 to 6.0.

In some embodiments, the carrier material of hydroxypropyl methylcellulose phthalate and the active ingredient are dissolved in the good solvent of dimethyl sulfoxide, the resulting solution is added to the poor solvent of water, and the pH is adjusted to 1.0 to 6.0.

In some embodiments, the method of the present disclosure comprises the following steps of:
a) dissolving the carrier material and the active ingredient (*R*)-4-amino-1-(1-(but-2-ynoyl)pyrrolidin-3-yl)-3-(4-(2,6-difluorophenoxy)phenyl)-1,6-dihydro-7*H-*pyrrolo[2,3-d]pyridazin-7-one or the pharmaceutically acceptable salt thereof in a good solvent, wherein the good solvent is at least one selected from the group consisting of dimethyl sulfoxide, N,N-dimethylformamide, N,N-dimethylacetamide, acetone, ethanol, tetrahydrofuran and methanol,
b) adding the solution obtained in step a) to a poor solvent, and adjusting the pH to 1.0 to 6.0, wherein the poor solvent is at least one selected from the group consisting of diethyl ether, *n*-hexane, petroleum ether and water.

In other embodiments, the method of the present disclosure comprises the following steps of:
a) dissolving the carrier material and the active ingredient (*R*)-4-amino-1-(1-(but-2-ynoyl)pyrrolidin-3-yl)-3-(4-(2,6-difluorophenoxy)phenyl)-1,6-dihydro-7*H-*pyrrolo[2,3-d]pyridazin-7-one or the pharmaceutically acceptable salt thereof in a good solvent, wherein the good solvent is at least one selected from the group consisting of dimethyl sulfoxide, N,N-dimethylformamide, N,N-dimethylacetamide, acetone, ethanol, tetrahydrofuran and methanol,
b) adding the solution obtained in step a) to a poor solvent, wherein the poor solvent is selected from the group consisting of water and a mixed solution of water with one or more of ether, *n*-hexane and petroleum ether, and the pH of the poor solvent is 1.0 to 6.0.

Furthermore, the method also comprises a step of filtration, washing or drying.

In other embodiments, the method of the present disclosure also comprises a step of spray drying.

In other embodiments, the particle size D90 of the solid dispersion is 50 µm to 2000 µm.

The particle size D90 of the solid dispersion provided in some embodiments can be selected from the group consisting of 200 µm, 210 µm, 220 µm, 230 µm, 240 µm, 250 µm, 260 µm, 270 µm, 280 µm, 290 µm, 300 µm, 310 µm, 320 µm, 330 µm, 340 µm, 350 µm, 360 µm, 370 µm, 380 µm, 390 µm, 400 µm, 410 µm, 420 µm, 430 µm, 440 µm, 450 µm, 460 µm, 470 µm, 480 µm, 490 µm, 500 µm, 510 µm, 520 µm, 530 µm, 540 µm, 550 µm, 560 µm, 570 µm, 580 µm, 590 µm, 600 µm, 610 µm, 620 µm, 630 µm, 640 µm, 650 µm, 660 µm, 670 µm, 680 µm, 690 µm, 700 µm, 710 µm, 720 µm, 730 µm, 740 µm, 750 µm, 760 µm, 770 µm, 780 µm, 790 µm, 800 µm, 810 µm, 820 µm, 830 µm, 840 µm, 850 µm, 860 µm, 870 µm, 880 µm, 890 µm, 900 µm, 910 µm, 920 µm, 930 µm, 940 µm, 950 µm, 960 µm, 970 µm, 980 µm, 990 µm, 1000 µm, 1010 µm, 1020 µm, 1030 µm, 1040 µm, 1050 µm, 1060 µm, 1070 µm, 1080 µm, 1090 µm, 1100 µm, 1110 µm, 1120 µm, 1130 µm, 1140 µm, 1150 µm, 1160 µm, 1170 µm, 1180 µm, 1190 µm, 1200 µm, 1210 µm, 1220 µm, 1230 µm, 1240 µm, 1250 µm, 1260 µm, 1270 µm, 1280 µm, 1290 µm, 1300 µm, 1310 µm, 1320 µm, 1330 µm, 1340 µm, 1350 µm, 1360 µm, 1370 µm, 1380 µm, 1390 µm, 1400 µm, 1410 µm, 1420 µm, 1430 µm, 1440 µm, 1450 µm, 1460 µm, 1470 µm, 1480 µm, 1490 µm, 1500 µm, 1510 µm, 1520 µm, 1530 µm, 1540 µm, 1550 µm, 1560 µm, 1570 µm, 1580 µm, 1590 µm, 1600 µm, 1610 µm, 1620 µm, 1630 µm, 1640 µm, 1650 µm, 1660 µm, 1670 µm, 1680 µm, 1690 µm, 1700 µm, 1710 µm, 1720 µm, 1730 µm, 1740 µm, 1750 µm, 1760 µm, 1770 µm, 1780 µm, 1790 µm, 1800 µm, 1810 µm, 1820 µm, 1830 µm, 1840 µm, 1850 µm, 1860 µm, 1870 µm, 1880 µm, 1890 µm, 1900 µm, 1910 µm, 1920 µm, 1930 µm, 1940 µm, 1950 µm, 1960 µm, 1970 µm, 1980 µm, 1990 µm, 2000 and any value between any two values, preferably 100 µm to 1500 µm, and more preferably 100 µm to 1000 µm.

Furthermore, the particle size D50 of the solid dispersion provided in some embodiments is 20 µm to 500 µm. Non-limiting examples include 50 µm, 60 µm, 70 µm, 80 µm, 90 µm, 100 µm, 110 µm, 120 µm, 130 µm, 140 µm, 150 µm, 160 µm, 170 µm, 180 µm, 190 µm, 200 µm, 210 µm, 220 µm, 230 µm, 240 µm, 250 µm, 260 µm, 270 µm, 280 µm, 290 µm, 300 µm, 310 µm, 320 µm, 330 µm, 340 µm, 350 µm, 360 µm, 370 µm, 380 µm, 390 µm, 400 µm, 410 µm, 420 µm, 430 µm, 440 µm, 450 µm, 460 µm, 470 µm, 480 µm, 490 µm, 500 µm or any value between any two values, and preferably 20 µm to 200 µm.

Furthermore, the particle size D10 of the solid dispersion provided in some embodiments is 1 µm to 100 µm. Non-limiting examples include 10 µm, 15 µm, 20 µm, 25 µm, 30 µm , 35 µm , 40 µm , 45 µm , 50 µm , 55 µm , 60 µm , 65 µm , 70 µm , 75 µm , 80 µm , 85 µm , 90 µm , 95 µm , 100 µm or any value between any two values, and preferably 5 µm to 50 µm.

Furthermore, the particle size D [4,3] of the solid dispersion provided in some embodiments is 100 µm to 800 µm. Non-limiting examples include 100 µm, 110 µm, 120 µm, 130 µm, 140 µm, 150 µm, 160 µm, 170 µm, 180 µm, 190 µm, 200 µm, 210 µm, 220 µm, 230 µm, 240 µm, 250 µm, 260 µm, 270 µm, 280 µm, 290 µm, 300 µm, 310 µm, 320 µm, 330 µm, 340 µm, 350 µm, 360 µm, 370 µm, 380 µm, 390 µm, 400 µm, 410 µm, 420 µm, 430 µm, 440 µm, 450 µm, 460 µm, 470 µm, 480 µm, 490 µm, 500 µm, 510 µm, 520 µm, 530 µm, 540 µm, 550 µm, 560 µm, 570 µm, 580 µm, 590 µm, 600 µm, 610 µm, 620 µm, 630 µm, 640 µm, 650 µm, 660 µm, 670 µm, 680 µm, 690 µm, 700 µm, 710 µm, 720 µm, 730 µm, 740 µm, 750 µm, 760 µm, 770 µm, 780 µm, 790 µm, 800 µm or any value between any two values, and preferably 150 µm to 600 µm.

The particle size D90 of the solid dispersion provided in the most preferred embodiment is 100 µm to 1000 µm, the particle size D50 of the same is 20 µm to 200 µm, and the particle size D10 of the same is 1 µm to 100 µm.

The particle size of the solid dispersion of the present disclosure refers to the particle size of the solid dispersion in the suspension obtained after the particles precipitated from the solvent, or the particle size of the solid dispersion after removing the solvent and drying. In some embodiments, the particle size refers to the particle size of the solid dispersion in the suspension obtained after the particles precipitated from the solvent.

In some embodiments, the active ingredient is in non-crystalline form.

In another aspect, the solid dispersion (SD) refers to a dispersion system in solid form formed by highly dispersing a drug in a solid carrier. The carrier material provides a dispersion system for the active ingredient. The higher the content of the carrier material, the easier it is for the active ingredient to transform from crystalline to amorphous, and the higher the bioavailability of the corresponding solid dispersion. In view of the balance between drug loading and bioavailability, the weight ratio of the carrier material to the active ingredient of the present disclosure can be 0.5:1 to 4:1. In some embodiments, the weight ratio can be 0.5:1, 0.6:1, 0.7:1, 0.8:1, 0.9:1, 1:1, 1.1:1, 1.2:1, 1.3:1, 1.4:1, 1.5:1, 1.6:1, 1.7:1, 1.8:1, 1.9:1, 2:1, 2.2:1, 2.4:1, 2.6:1, 2.8:1, 3:1, 3.2:1, 3.4:1, 3.6:1, 3.8:1, 4:1 or any value between any two values, and preferably 0.8:1 to 3:1.

In the solid dispersion provided in some embodiments, the carrier material is at least one selected from the group consisting of hydroxypropyl methylcellulose acetate succinate, polyvinylpyrrolidone, hydroxypropyl methylcellulose phthalate and polyvinylpyrrolidone.

The solid dispersion provided in some preferred embodiments comprises polyvinylpyrrolidone and the active ingredient (*R*)-4-amino-1-(1-(but-2-ynoyl)pyrrolidin-3-yl)-3-(4-(2,6-difluorophenoxy)phenyl)-1,6-dihydro-7*H*-pyrrolo[2,3-d]pyridazin-7-one or the pharmaceutically acceptable salt thereof.

Furthermore, the solid dispersion of the present disclosure consists of the active ingredient (*R*)-4-amino-1-(1-(but-2-ynoyl)pyrrolidin-3-yl)-3-(4-(2,6-difluorophenoxy)phenyl)-1,6-dihydro-7*H*-pyrrolo[2,3-d]pyridazin-7-one or the pharmaceutically acceptable salt thereof and the carrier material.

The present disclosure also provides a solid dispersion prepared by the above method.

The present disclosure also provides a solid dispersion comprising the active ingredient (*R*)-4-amino-1-(1-(but-2-ynoyl)pyrrolidin-3-yl)-3-(4-(2,6-difluorophenoxy)phenyl)-1,6-dihydro-7*H*-pyrrolo[2,3-d]pyridazin-7-one or the pharmaceutically acceptable salt thereof and a carrier material, wherein the particle size D90 of the solid dispersion is 50 µm to 2000 µm.

The particle size D90 of the solid dispersion provided in some embodiments can be selected from the group consisting of 200 µm, 210 µm, 220 µm, 230 µm, 240 µm, 250 µm, 260 µm, 270 µm, 280 µm, 290 µm, 300 µm, 310 µm, 320 µm, 330 µm, 340 µm, 350 µm, 360 µm, 370 µm, 380 µm, 390 µm, 400 µm, 410 µm, 420 µm, 430 µm, 440 µm, 450 µm, 460 µm, 470 µm, 480 µm, 490 µm, 500 µm, 510 µm, 520 µm, 530 µm, 540 µm, 550 µm, 560 µm, 570 µm, 580 µm, 590 µm, 600 µm, 610 µm, 620 µm, 630 µm, 640 µm, 650 µm, 660 µm, 670 µm, 680 µm, 690 µm, 700 µm, 710 µm, 720 µm, 730 µm, 740 µm, 750 µm, 760 µm, 770 µm, 780 µm, 790 µm, 800 µm, 810 µm, 820 µm, 830 µm, 840 µm, 850 µm, 860 µm, 870 µm, 880 µm, 890 µm, 900 µm, 910 µm, 920 µm, 930 µm, 940 µm, 950 µm, 960 µm, 970 µm, 980 µm, 990 µm, 1000 µm, 1010 µm, 1020 µm, 1030 µm, 1040 µm, 1050 µm, 1060 µm, 1070 µm, 1080 µm, 1090 µm, 1100 µm, 1110 µm, 1120 µm, 1130 µm, 1140 µm, 1150 µm, 1160 µm, 1170 µm, 1180 µm, 1190 µm, 1200 µm, 1210 µm, 1220 µm, 1230 µm, 1240 µm, 1250 µm, 1260 µm, 1270 µm, 1280 µm, 1290 µm, 1300 µm, 1310 µm, 1320 µm, 1330 µm, 1340 µm, 1350 µm, 1360 µm, 1370 µm, 1380 µm, 1390 µm, 1400 µm, 1410 µm, 1420 µm, 1430 µm, 1440 µm, 1450 µm, 1460 µm, 1470 µm, 1480 µm, 1490 µm, 1500 µm, 1510 µm, 1520 µm, 1530 µm, 1540 µm, 1550 µm, 1560 µm, 1570 µm, 1580 µm, 1590 µm, 1600 µm, 1610 µm, 1620 µm, 1630 µm, 1640 µm, 1650 µm, 1660 µm, 1670 µm, 1680 µm, 1690 µm, 1700 µm, 1710 µm, 1720 µm, 1730 µm, 1740 µm, 1750 µm, 1760 µm, 1770 µm, 1780 µm, 1790 µm, 1800 µm, 1810 µm, 1820 µm, 1830 µm, 1840 µm, 1850 µm, 1860 µm, 1870 µm, 1880 µm, 1890 µm, 1900 µm, 1910 µm, 1920 µm, 1930 µm, 1940 µm, 1950 µm, 1960 µm, 1970 µm, 1980 µm, 1990 µm, 2000 and any value between any two values, preferably 100 µm to 1500 µm, and more preferably 100 µm to 1000 µm.

Furthermore, the particle size D50 of the solid dispersion provided in some embodiments is 20 µm to 500 µm. Non-limiting examples include 50 µm, 60 µm, 70 µm, 80 µm, 90 µm, 100 µm, 110 µm, 120 µm, 130 µm, 140 µm, 150 µm, 160 µm, 170 µm, 180 µm, 190 µm, 200 µm, 210 µm, 220 µm, 230 µm, 240 µm, 250 µm, 260 µm, 270 µm, 280 µm, 290 µm, 300 µm, 310 µm, 320 µm, 330 µm, 340 µm, 350 µm, 360 µm, 370 µm, 380 µm, 390 µm, 400 µm, 410 µm, 420 µm, 430 µm, 440 µm, 450 µm, 460 µm, 470 µm, 480 µm, 490 µm, 500 µm or any value between any two values, and preferably 20 µm to 200 µm.

Furthermore, the particle size D10 of the solid dispersion provided in some embodiments is 1 µm to 100 µm. Non-limiting examples include 10 µm, 15 µm, 20 µm, 25 µm, 30 µm, 35 µm, 40 µm, 45 µm, 50 µm, 55 µm, 60 µm, 65 µm, 70 µm, 75 µm, 80 µm, 85 µm, 90 µm, 95 µm, 100 µm or any value between any two values, and preferably 5 µm to 50 µm.

Furthermore, the particle size D [4,3] of the solid dispersion provided in some embodiments is 100 µm to 800 µm. Non-limiting examples include 100 µm, 110 µm, 120 µm, 130 µm, 140 µm, 150 µm, 160 µm, 170 µm, 180 µm, 190 µm, 200 µm, 210 µm, 220 µm, 230 µm, 240 µm, 250 µm, 260 µm, 270 µm, 280 µm, 290 µm, 300 µm, 310 µm, 320 µm, 330 µm, 340 µm, 350 µm, 360 µm, 370 µm, 380 µm, 390 µm, 400 µm, 410 µm, 420 µm, 430 µm, 440 µm, 450 µm, 460 µm, 470 µm, 480 µm, 490 µm, 500 µm, 510 µm, 520 µm, 530 µm, 540 µm, 550 µm, 560 µm, 570 µm, 580 µm, 590 µm, 600 µm, 610 µm, 620 µm, 630 µm, 640 µm, 650 µm, 660 µm, 670 µm, 680 µm, 690 µm, 700 µm, 710 µm, 720 µm, 730 µm, 740 µm, 750 µm, 760 µm, 770 µm, 780 µm, 790 µm, 800 µm or any value between any two values, and preferably 150 µm to 600 µm.

The particle size D90 of the solid dispersion provided in the most preferred embodiment is 100 µm to 1000 µm, the particle size D50 of the same is 20 µm to 200 µm, and the particle size D10 of the same is 1 µm to 100 µm.

In some embodiments, the active ingredient is in non-crystalline form.

In view of the balance between drug loading and bioavailability, the weight ratio of the carrier material to the active ingredient of the present disclosure can be 0.5:1 to 4:1. In some embodiments, the weight ratio can be 0.5:1, 0.6:1, 0.7:1, 0.8:1, 0.9:1, 1:1, 1.1:1, 1.2:1, 1.3:1, 1.4:1, 1.5:1, 1.6:1, 1.7:1, 1.8:1, 1.9:1, 2:1, 2.2:1, 2.4:1, 2.6:1, 2.8:1, 3:1, 3.2:1, 3.4:1, 3.6:1, 3.8:1, 4:1 or any value between any two values, and preferably 0.8:1 to 3:1.

In the solid dispersion provided in some embodiments, the carrier material is at least one selected from the group consisting of hydroxypropyl methylcellulose acetate succinate, polyvinylpyrrolidone, hydroxypropyl methylcellulose phthalate and polyvinylpyrrolidone.

The solid dispersion provided in some preferred embodiments comprises polyvinylpyrrolidone and the active ingredient (*R*)-4-amino-1-(1-(but-2-ynoyl)pyrrolidin-3-yl)-3-(4-(2,6-difluorophenoxy)phenyl)-1,6-dihydro-7*H*-pyrrolo[2,3-d]pyridazin-7-one or the pharmaceutically acceptable salt thereof.

Furthermore, the solid dispersion of the present disclosure is consisting of the active ingredient (R)-4-amino-1-(1-(but-2-ynoyl)pyrrolidin-3-yl)-3-(4-(2,6-difluorophenoxy)phenyl)-1,6-dihydro-7*H*-pyrrolo[2,3-d]pyridazin-7-one or the pharmaceutically acceptable salt thereof and the carrier material.

In another aspect, the present disclosure provides a solid formulation comprising the above solid dispersion or the solid dispersion prepared by the above method and optionally a pharmaceutically acceptable excipient, wherein the excipient is at least one selected from the group consisting of disintegrant, filler, binder and lubricant. The solid formulation can be a tablet, pill, granule, capsule, or the like.

In some embodiments, the content of the active ingredient is 8 to 40% by weight, relative to the weight of the pharmaceutical composition. The content of the active ingredient can be 8, 9, 10, 11, 12, 13, 14, 15, 16, 17, 18, 19, 20, 21, 22, 23, 24, 25, 26, 27, 28, 29, 30, 35, 40% or any value between any two values, and preferably 15 to 25% by weight, relative to the weight of the pharmaceutical composition.

In other embodiments, the amount (weight or mass) of the active ingredient is 10 to 500 mg. The the amount (weight or mass) of the active ingredient can be 200 mg, 190 mg, 180 mg, 170 mg, 160 mg, 150 mg, 140 mg, 130 mg, 120 mg, 110 mg, 100 mg, 95 mg, 75 mg, 50 mg, 25 mg, 15 mg, 10 mg or any value between any two values, and preferably 200 mg, 100 mg or 25 mg.

The disintegrant of the present disclosure is known or determinable by those skilled in the art, and seleceted, but not limited to, at least one of croscarmellose sodium, crospovidone, sodium carboxymethyl starch, starch, pregelatinized starch and alginic acid. Preferably, the disintegrant is present in an amount of 1 to 20% by weight, relative to the weight of the pharmaceutical composition. Non-limiting examples include 1.0, 1.5,2,2.5, 3, 3.5, 4, 4.5, 5, 5.5, 6, 6.5, 7, 7.5, 8, 8.5, 9, 9.5, 10, 11, 12, 13, 14, 15, 16, 17, 18, 19, 20% or any value between any two values, and preferably 5 to 15% by weight, relative to the weight of the pharmaceutical composition.

The binder of the present disclosure is known or determinable by those skilled in the art, and includes, but not limited to, at least one of polyvinylpyrrolidone, starch, methyl cellulose, carboxy cellulose, hydroxypropyl cellulose, hydroxypropyl methyl cellulose and alginate, preferably at least one of polyvinylpyrrolidone (trade name K30) and hydroxypropyl cellulose. More preferably, the binder is present in an amount of 0.5 to 10% by weight, relative to the weight of the pharmaceutical composition. Non-limiting examples include 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, 10% or any value between any two values by weight, relative to the weight of the pharmaceutical composition.

The lubricant of the present disclosure is known or determinable by those skilled in the art, and includes, but not limited to, at least one of magnesium stearate, stearic acid, palmitic acid, calcium stearate, talc, carnauba wax and sodium stearyl fumarate. Preferably, the lubricant is present in an amount of 0.1 to 5% by weight, relative to the weight of the pharmaceutical composition. Non-limiting examples include 0.1, 0.2, 0.3, 0.4, 0.5, 0.6, 0.7, 0.8, 0.9%, 1%, 1.5%, 2%, 2.5%, 3%, 3.5%, 4%, 4.5, 5% or any value between any two values, and preferably 0.1 to 2% by weight, relative to the weight of the pharmaceutical composition.

In some embodiments, the solid formulation of the present disclosure comprises:
1) 10 mg to 500 mg of the active ingredient,
2) 5 to 15% by weight of the disintegrant,
3) 30 to 90% by weight of the filler,
4) 0.5 to 10% by weight of the binder,
5) 0.1 to 5% by weight of the lubricant.

Furthermore, the dissolution rate is determined according to the second method (paddle method) of the dissolution rate test described in general rule of volume IV of Chinese Pharmacopoeia 2015 Edition, using 0.15% aqueous solution of SDS as a dissolution medium, at 37±0.5° C, and at a paddle speed of 50 rpm. The dissolution rate (%)in 45 minutes of the active ingredient in the solid formulation of the present disclosure is 85% or greater, and can be greater than or equal to 85, 86, 87, 88, 89, 90, 91, 92, 93, 94, 95, 96, 97, 98, 99, 100%, and preferably 90% or greater. Furthermore, the dissolution rate (%) in 15 minutes of the active ingredient in the solid formulation is 70% or greater, and can be greater than or equal to 70, 71, 72, 73, 74, 75, 76, 77, 78, 79, 80, 81, 82, 83, 84, 85, 86, 87, 88, 89, 90, 91, 92, 93, 94, 95%. The solid formulation dissolves rapidly and completely, and has a good bioavailability. The preparation process of the solid formulation is simple, and suitable for large-scale production.

Furthermore, the present disclosure also provides a method for preparing the above solid formulation comprising the steps of pulverizing the solid dispersion, mixing well with the filler and/or disintegrant required for molding a pharmaceutical composition, adding the binder, subjecting to wet granulation or dry granulation, drying the resulting granules, screening by a sieve, milling, mixing well with the lubricant, and preparing into pills or granules or compressing into tablets or filling into capsules; or the solid dispersion can also be added directly into a capsule with suitable auxiliary materials or be compressed into tablets. The resulting granules or raw tablets or capsules can be further coated as needed.

The present disclosure also provides a use of the solid dispersion or solid formulation of the present disclosure in the preparation of a medicament for the treatment of conditions or diseases mediated by protein tyrosine kinase. In some embodiments, the condition or disease is a cancer or autoimmune disease. In some embodiments, the cancer is a B cell malignancy selected from the group consisting of chronic lymphocytic leukemia (CLL), mantle cell lymphoma (MCL), diffuse large B-cell lymphoma (DLBCL), multiple myeloma (MM), follicular lymphoma (FL), marginal zone lymphoma and Waldenstrom's macroglobulinemia (WM). In some embodiments, the autoimmune disease is rheumatoid arthritis or systemic lupus erythematosus.

The active ingredient (*R*)-4-amino-1-(1-(but-2-ynoyl)pyrrolidin-3-yl)-3-(4-(2,6-difluorophenoxy)phenyl)-1,6-dihydro-7*H*-pyrrolo[2,3-d]pyridazin-7-one of the present disclosure can be formed into a pharmaceutically acceptable salt with an acid. The acid is known or determinable by those skilled in the art and includes, but is not limited to, hydrochloric acid, methanesulfonic acid, fumaric acid, trifluoroacetic acid and phosphoric acid.

The expression "relative to the weight of the pharmaceutical composition" of the present disclosure means that the calculation of the range of the amount of the active ingredient or other kinds of pharmaceutical auxiliary materials is based on the weight of the tablet core without a coating agent.

The "good solvent" and "poor solvent (weak solvent)" of the present disclosure are classified according to the solubility of the active ingredient. In general, poor solvent and good solvent are classified by the solubility of 20 mg/ml. If a solvent has a solubility of the active ingredient lower than about 20 mg/ml, then the solvent is a poor solvent. Correspondingly, if a solvent has a solubility of the active ingredient higher than about 20 mg/ml, then the solvent is a good solvent.

The term "D10" of the present disclosure refers to the corresponding particle size when the cumulative particle size distribution percentage of a sample reaches 10%. The term "D50" refers to the corresponding particle size when the cumulative particle size distribution percentage of a sample reaches 50%. The term "D90" refers to the corresponding particle size when the cumulative particle size distribution percentage of a sample reaches 90%. D[4,3] represents the "fourth moment/volume" average diameter, also known as the volume (or weight) average diameter. For those skilled in the art, there is a certain degree of error in particle size measurement. In general, plus or minus 10% are within a reasonable error range. D10, D50, D90 and D[4,3] have a certain degree of error variation depending on the context in which they are used, and the error variation does not exceed plus or minus 10%.

HPLC detection conditions of the present disclosure:
Octadecylsilane bonded silica is used as the filler (Waters Symmetry C18 colume); 0.01 mol/L potassium dihydrogen phosphate buffer solution and acetonitrile are used as the mobile phase and eluent; the detection wavelength is 210 nm.

The pharmaceutical auxiliary materials and reagents, such as hydroxypropyl methylcellulose acetate succinate, are commercially available. (*R*)-4-Amino-1-(1-(but-2-ynoyl)pyrrolidin-3-yl)-3-(4-(2,6-difluorophenoxy)phenyl)-1,6-dihydro-7H-pyrrolo[2,3-d]pyridazin-7-one (compound A) or a pharmaceutically acceptable salt thereof can be prepared according to the method described in Example 109 of WO2016007185.

### DESCRIPTION OF THE DRAWINGS

The above and other objects and features of the present disclosure will become apparent with reference to the following drawings, which respectively represent:
Figure 1: SEM image of the sample of Example 3
Figure 2: SEM image of the sample of Example 4

### DETAILED DESCRIPTION

The present disclosure will be further described in detail with reference to the following examples and experimental examples. These examples and experimental examples are for illustrative purposes only, and should not be considered as limiting the scope of the present disclosure.

### Example 1:

1 g of (*R*)-4-amino-1-(1-(but-2-ynoyl)pyrrolidin-3-yl)-3-(4-(2,6-difluorophenoxy)phenyl)-1,6-dihydro-7*H*-pyrrolo[2,3-d]pyridazin-7-one (abbreviated as compound A) and 1 g of hydroxypropyl methylcellulose acetate succinate (HPMC-AS) were added to 15 ml of dimethylacetamide, and stirred to dissolve. The resulting solution was added dropwise into 100 ml of water at a rate of 4 g/min or 2 g/min, and stirred for about 1 hour. The resulting suspension was filtered. Agglomeration was observed initially, filtration (or suction filtration) was difficult, and the suction filtrate appeared milky.

### Example 2:

1 g of compound A and 1 g of hydroxypropyl methylcellulose acetate succinate (HPMC-AS) were added to 15 ml of dimethylacetamide, and stirred to dissolve. The resulting solution was added dropwise at a rate of 4 g/min into 100 ml of water with different pH (shown in Table 1), and stirred for about 1 hour. The resulting suspension was filtered to obtain solid. The observed phenomena are as follows:

**Table 1**

| Experimental example | pH | Acid type | Powder appearance | Phenomenon | Active ingredient content% | XRPD |
|---|---|---|---|---|---|---|
| 1 | 2 | 98% sulfuric acid | Fluffy | Evenly dispersed, easy to filter, and the filtrate was clear | 47.39% | Amorphous |
| 2 | 2 | 85% phosphoric acid | Fluffy | Evenly dispersed, easy to filter, and the filtrate was clear | 50.88% | Amorphous |
| 3 | 3 | Glacial acetic acid | Fluffy | Evenly dispersed, easy to filter, and the filtrate was clear | 47.25% | Amorphous |
| 4 | 3 | 98% sulfuric acid | Fluffy | Evenly dispersed, easy to filter, and the filtrate was clear | 50.75% | Amorphous |
| 5 | 3 | 36.5% hydrochloric acid | Tight | Evenly dispersed, easy to filter, and the filtrate was clear | 46.35% | Amorphous |

Conclusion: The solid dispersion prepared with phosphoric acid or sulfuric acid has low residual ion content. For example, when using phosphoric acid, the phosphorus content in the obtained solid dispersion is about 20 µg/g; and when using sulfuric acid, the sulfur content in the obtained solid dispersion is 10 µg/g.

The samples obtained in Experimental Examples 1, 4 and 5 were placed at 93%, 60°C, 40°C/75%RH, 25°C/60%RH conditions respectively to investigate the physical and chemical stability. Data are shown as follows:

| Conditions | | Day 7 | | |
|---|---|---|---|---|
| Humidity (%) | Temperature (°C) | 1 | 4 | 5 |
| 60 | 25 | 99.42% | 99.40% | 99.37% |
| 75 | 40 | 99.41% | 99.38% | 99.38% |
| 93 | / | 99.42% | 99.40% | 99.39% |
| / | 60 | 99.42% | 99.39% | 99.38% |
| Conditions | | Day 14 | | |

| Humidity (%) | Temperature (°C) | 1 | 4 | 5 |
|---|---|---|---|---|
| 60 | 25 | 99.41% | 99.39% | 99.43% |
| 75 | 40 | 99.40% | 99.40% | 99.40% |
| 93 | / | 99.39% | 99.41% | 99.42% |
| / | 60 | 99.39% | 99.40% | 99.41% |

### Example 3:

1 g of compound A and 1 g of HPMC-AS were dissolved in 15 ml of N,N-dimethylacetamide. The resulting solution was added dropwise at a rate of 4 g/min into 100 ml of water solution (adjusted to pH=2 with 36.5% hydrochloric acid), and stirred for 30 minutes. The resulting suspension was filtered, and the filter cake was rinsed with water. The resulting solid was dried overnight at 40°C, and subjected to SEM determination.

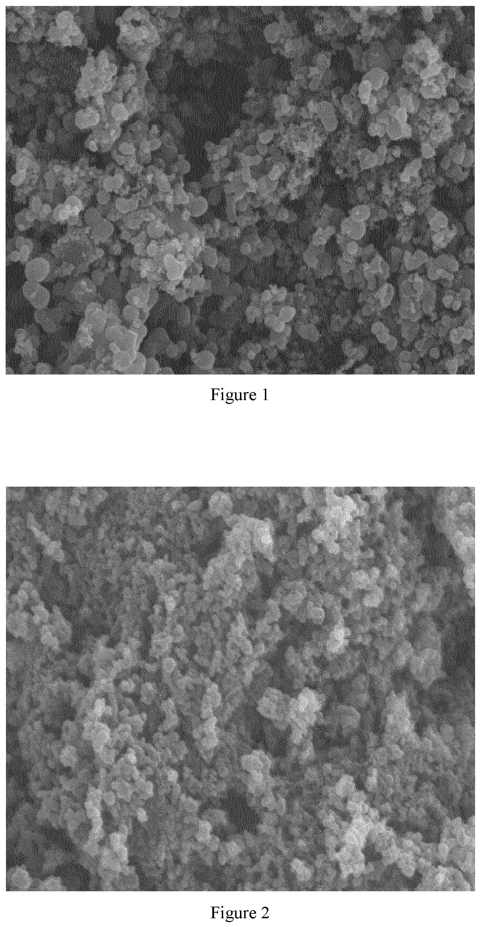

SEM result shows that the obtained sample is microsphere-like and has uniformly distribution, see Figure 1.

### Example 4:

1 g of compound A and 1 g of HPMC-AS were dissolved in 15 ml of N,N-dimethylacetamide. The resulting solution was added dropwise at a rate of 4 g/min into 100 ml of water and stirred. The sample obtained with water as the dispersion medium was used for particle size determination. The resulting suspension was filtered, the suction filtrate appeared milky, and the filter cake was rinsed with water. The resulting solid was dried overnight at 40°C, and subjected to SEM determination.

Particle size of the sample obtained with water as the dispersion medium: D10=7.6 µm, D50=27.2 µm, D90=86 µm, D [4,3]=39.4 µm.

SEM result shows that the obtained sample is granular with uneven particle size, see Figure 2.

### Example 5:

A certain amount of compound A and HPMC-AS-LF were weighed and dissolved in 15 ml of N,N-dimethylacetamide (DMAC). The resulting solution was added dropwise at a rate of 4 g/min into 100 ml of water (adjusted to pH=2 with 36.5% hydrochloric acid) and stirred. The specific parameters are shown in the table below:

| Test Example | Stirring method | Compound (g) | HPMC-AS (g) | pH value | Temperature (°C) | Stirring rate (rpm) |
|---|---|---|---|---|---|---|
| 6 | Mechanical stirring | 1 | 1 | 2 | 25 | 300 |
| 7 | Mechanical stirring | 1 | 1 | 2 | 15 | 300 |
| 8 | Mechanical stirring | 1 | 1 | 2 | 10 | 300 |
| 9 | Mechanical stirring | 1 | 1 | 2 | 5 | 300 |
| 10 | Mechanical | 1 | 1 | 2 | 0 | 300 |
| | stirring | | | | | |

The samples obtained with water as the dispersion medium were subjected to particle size determination respectively. The specific data are as follows:

| Test Example | D10 (µm) | D50 (µm) | D90 (µm) | D [4,3] (µm) |
|---|---|---|---|---|
| 6 | 19.9 | 165 | 497 | 223 |
| 7 | 21.6 | 209 | 746 | 349 |
| 8 | 17.5 | 151 | 477 | 203 |
| 9 | 19.3 | 163 | 460 | 204 |
| 10 | 33.1 | 268 | 1430 | 501 |

### Example 6:

A solid dispersion comprising compound A and hydroxypropyl methylcellulose acetate succinate was prepared by the method of Test Example 9. A prescription amount of the solid dispersion, lactose, microcrystalline cellulose and croscarmellose sodium were weighed according to the formulation specified as follows. The mixture was poured into a granulating tank, mixed well, and polyvinylpyrrolidone was added as the binder to prepare granules. The wet and soft material was wet-milled and dried, and then the dry granules (water content less than 3%) were dry-milled. Extragranular auxiliary materials were added, and mixed well with the granules. The resulting total mixed granules were compressed into tablets. Specific prescription ratios are shown in Table 2.

**Table 2**

| Ingredients | Experimental Example (mg/tablet) | |
|---|---|---|
| | 11 | 12 |
| Compound A | 100 | 100 |
| Hydroxypropyl methylcellulose acetate succinate | 100 | 200 |
| Lactose monohydrate | 175 | 175 |
| Microcrystalline cellulose 101 | 60 | 60 |
| Croscarmellose sodium (intragranularly) | 25 | 25 |
| Polyvinylpyrrolidone K30 | 20 | 20 |
| Croscarmellose sodium (extragranularly) | 15 | 15 |
| Magnesium stearate | 5.0 | 5.6 |
| Total (mg) | 500 | 600 |

### Dissolution Test

The dissolution rates of the tablets of Experimental Examples 11 and 12 were determined according to the second method (paddle method) of the dissolution rate test described in general rule of volume IV of Chinese Pharmacopoeia 2015 Edition. The dissolution test was carried out using 1000 ml of 0.15% aqueous solution of SDS as a dissolution medium, at 37±0.5°C, and at a paddle speed of 50 rpm.

**Table 3**

| Time (min) | Dissolution rate (%) | |
|---|---|---|
| | 11 | 12 |
| 5 | 60.3 | 63.2 |
| 15 | 80.2 | 82.9 |
| 45 | 97.8 | 98.2 |

## Claims

1. A method for preparing a solid dispersion, comprising the steps of dissolving a carrier material and the active ingredient (*R*)-4-amino-1-(1-(but-2-ynoyl)pyrrolidin-3-yl)-3-(4-(2,6-difluorophenoxy)phenyl)-1,6-dihydro-7*H*-pyrrolo[2,3-d]pyridazin-7-one or a pharmaceutically acceptable salt thereof in a good solvent to obtain a solution, adding the resulting solution to a poor solvent, and adjusting the pH to 1.0 to 6.0, and preferably pH=2.0 to 4.0; or, comprising the steps of dissolving a carrier material and the active ingredient in a good solvent to obtain a solution, and adding the resulting solution to a poor solvent, wherein the pH of the poor solvent is 1.0 to 6.0, and preferably pH=2.0 to 4.0.

2. The method according to claim 1, wherein the good solvent is at least one selected from the group consisting of dimethyl sulfoxide, N,N-dimethylformamide, N,N-dimethylacetamide, acetone, ethanol, tetrahydrofuran and methanol, and preferably N,N-dimethylformamide or N,N-dimethylacetamide.

3. The method according to claim 1 or 2, wherein the poor solvent is at least one selected from the group consisting of diethyl ether, *n*-hexane, petroleum ether and water.

4. The method according to any one of claims 1 to 3, wherein the reagent used to adjust pH is at least one selected from the group consisting of hydrochloric acid, sulfuric acid, acetic acid and phosphoric acid, and preferably sulfuric acid or hydrochloric acid.

5. The method according to any one of claims 1 to 4, wherein the addition to the poor solvent is accompanied by a stirring process, the stirring rate is selected from the group consisting of 20 to 1000 rpm, and preferably 100 to 600 rpm.

6. The method according to any one of claims 1 to 5, wherein the temperature for the precipitation of solid is selected from the group consisting of 0 to 40°C.

7. The method according to any one of claims 1 to 6, comprising:
method 1:
a) dissolving the carrier material and the active ingredient (*R*)-4-amino-1-(1-(but-2-ynoyl)pyrrolidin-3-yl)-3-(4-(2,6-difluorophenoxy)phenyl)-1,6-dihydro-7*H-*pyrrolo[2,3-d]pyridazin-7-one or the pharmaceutically acceptable salt thereof in a good solvent, wherein the good solvent is at least one selected from the group consisting of dimethyl sulfoxide, N,N-dimethylformamide, N,N-dimethylacetamide, acetone, ethanol, tetrahydrofuran and methanol,
b) adding the solution obtained in step a) to a poor solvent, and adjusting the pH to 1.0 to 6.0, wherein the poor solvent is at least one selected from the group consisting of diethyl ether, *n*-hexane, petroleum ether and water;
or, method 2:
a) dissolving the carrier material and the active ingredient (*R*)-4-amino-1-(1-(but-2-ynoyl)pyrrolidin-3-yl)-3-(4-(2,6-difluorophenoxy)phenyl)-1,6-dihydro-7*H-*pyrrolo[2,3-d]pyridazin-7-one or the pharmaceutically acceptable salt thereof in a good solvent, wherein the good solvent is at least one selected from the group consisting of dimethyl sulfoxide, N,N-dimethylformamide, N,N-dimethylacetamide, acetone, ethanol, tetrahydrofuran and methanol,
b) adding the solution obtained in step a) to a poor solvent, wherein the poor solvent is selected from the group consisting of water and a mixed solution of water with one or more of ether, *n*-hexane and petroleum ether, and the pH of the poor solvent is 1.0 to 6.0.

8. The method according to any one of claims 1 to 7, wherein the carrier material is at least one selected from the group consisting of hydroxypropyl methylcellulose acetate succinate, polyvinylpyrrolidone, hydroxypropyl methylcellulose phthalate and polyvinylpyrrolidone.

9. The method according to any one of claims 1 to 8, wherein the weight ratio of the carrier material to the active ingredient is 0.5:1 to 4:1, and preferably 0.8:1 to 3:1.

10. The method according to any one of claims 1 to 9, wherein the solid dispersion is consisting of the active ingredient (*R*)-4-amino-1-(1-(but-2-ynoyl)pyrrolidin-3-yl)-3-(4-(2,6-difluorophenoxy)phenyl)-1,6-dihydro-7*H*-pyrrolo[2,3-d]pyridazin-7-one or the pharmaceutically acceptable salt thereof and the carrier material.

11. The method according to any one of claims 1 to 10, wherein the particle size D90 of the solid dispersion is 50 µm to 2000 µm, preferably 100 µm to 1500 µm, and more preferably 100 µm to 1000 µm.

12. The method according to any one of claims 1 to 11, wherein the particle size D50 of the solid dispersion is 20 µm to 500 µm, and preferably 20 µm to 200 µm.

13. The method according to any one of claims 1 to 12, wherein the particle size D10 of the solid dispersion is 1 µm to 100 µm, and preferably 5 µm to 50 µm.

14. A method for preparing a solid dispersion, comprising the steps of dissolving a carrier material and an active ingredient in a good solvent to obtain a solution, adding the resulting solution to a poor solvent, and adjusting the pH to 1.0 to 6.0, and preferably pH=2.0 to 4.0; or, comprising the steps of dissolving a carrier material and an active ingredient in a good solvent to obtain a solution, and adding the resulting solution to a poor solvent, wherein the pH of the poor solvent is 1.0 to 6.0, and preferably 2.0 to 4.0.

15. A solid dispersion comprising the active ingredient (*R*)-4-amino-1-(1-(but-2-ynoyl)pyrrolidin-3-yl)-3-(4-(2,6-difluorophenoxy)phenyl)-1,6-dihydro-7*H*-pyrrolo[2,3-d]pyridazin-7-one or the salt thereof and a carrier material, wherein the particle size D90 of the solid dispersion is 50 µm to 2000 µm, preferably 100 µm to 1500 µm, and more preferably 100 µm to 1000 µm.

16. The solid dispersion according to claim 15, wherein the particle size D50 of the solid dispersion is 20 µm to 500 µm, and preferably 20 µm to 200 µm.

17. The solid dispersion according to claim 15 or 16, wherein the particle size D10 of the solid dispersion is 1 µm to 100 µm, and preferably 5 µm to 50 µm.

18. The solid dispersion according to any one of claims 15 to 17, wherein the carrier material is at least one selected from the group consisting of hydroxypropyl methylcellulose acetate succinate, polyvinylpyrrolidone, hydroxypropyl methylcellulose phthalate and polyvinylpyrrolidone.

19. The solid dispersion according to any one of claims 15 to 18, wherein the weight ratio of the carrier material to the active ingredient is 0.5:1 to 4:1, and preferably 0.8:1 to 3:1.

20. The solid dispersion according to any one of claims 15 to 19, wherein the solid dispersion is consisting of the active ingredient (*R*)-4-amino-1-(1-(but-2-ynoyl)pyrrolidin-3-yl)-3-(4-(2,6-difluorophenoxy)phenyl)-1,6-dihydro-7*H*-pyrrolo[2,3-d]pyridazin-7-one or the pharmaceutically acceptable salt thereof and the carrier material.

21. A solid formulation comprising the solid dispersion according to any one of claims 15 to 20 or the solid dispersion prepared by the method according to any one of claims 1 to 14 and optionally at least one excipient selected from the group consisting of disintegrant, filler, binder and lubricant.

22. The solid formulation according to claim 21, wherein the disintegrant is at least one selected from the group consisting of croscarmellose sodium, crospovidone, sodium carboxymethyl starch, starch, pregelatinized starch and alginic acid, and preferably the disintegrant is present in an amount of 1 to 20% by weight, relative to the weight of the pharmaceutical composition; the binder is at least one selected from the group consisting of polyvinylpyrrolidone, starch, methyl cellulose, carboxy cellulose, hydroxypropyl cellulose, hydroxypropyl methyl cellulose and alginate, and preferably the binder is present in an amount of 0.5 to 10% by weight, relative to the weight of the pharmaceutical composition; and the lubricant is at least one selected from the group consisting of magnesium stearate, stearic acid, palmitic acid, calcium stearate, talc, carnauba wax and sodium stearyl fumarate, and preferably the lubricant is present in an amount of 0.1 to 5% by weight, relative to the weight of the pharmaceutical composition.

23. The solid formulation according to claim 21 or 22, comprising:
1) 10 mg to 500 mg of the active ingredient,
2) 5 to 15% by weight of the disintegrant,
3) 30 to 90% by weight of the filler,
4) 0.5 to 10% by weight of the binder,
5) 0.1 to 5% by weight of the lubricant.

24. The solid formulation according to any one of claims 21 to 23, wherein the dissolution rate of the active ingredient determined according to the second method (paddle method) of the dissolution rate test described in general rule of volume IV of Chinese Pharmacopoeia 2015 Edition, using 0.15% aqueous solution of SDS as a dissolution medium, at 37±0.5°C, and at a paddle speed of 50 rpm, is no less than 85%, and preferably no less than 90% in 45 minutes , .
